# EUROPEAN PATENT APPLICATION

(11) **EP 3 871 711 A1**
(43) Date of publication of application: **01.09.2021**
(21) Application number: 21159282.9
(22) Date of filing: 25.02.2021
(51) Int. Cl.: A61M 5/31, A61M 5/315

(54) **APPARATUS FOR OPERATING A MEDICAL SYRINGE**

(30) Priority: 26.02.2020 DE 102020105039
(71) Applicant: Ioannidis, Sotirios, 54352 Thessaloniki (GR)
(72) Inventor: Ioannidis, Sotirios, 54352 Thessaloniki (GR)
(74) Representative: DREISS Patentanwälte PartG mbB

(57) **Abstract**

An apparatus (2) for operating a medical syringe (4) is provided, the apparatus (2) comprising: a first retaining section (100) configured to receive and hold a barrel section (10) of the medical syringe (4); a second retaining section (200) configured to receive and hold a plunger section (20) of the medical syringe (20); and an actuator (300) configured to axially displace the first retaining section (100) and the second retaining section (200) in relation to each other and along an actuating axis (ax).

## Description

Examples provide advantages in the technical field of medical syringes.

A medical syringe is a medical instrument used to deliver drugs (injection), to withdraw body fluids (puncture) or to extract body tissue (biopsy), to infuse fluid and food, to cleanse (for example, wounds) and to deliver injectable fillers (injection).

The problems of the prior art are resolved by an apparatus according to claim 1. Further advantageous features can be found in the other claims, in the following description and in the drawings.

According to an aspect of the description, an apparatus for operating a medical syringe is provided. The apparatus comprises: a first retaining section configured to receive and hold a barrel section of the medical syringe; a second retaining section configured to receive and hold a plunger section of the medical syringe; and an actuator configured to axially displace the first retaining section and the second retaining section in relation to each other and along an actuating axis.

The handling apparatus for the syringe advantageously simplifies usage of the syringe. The medical practitioner is enabled to safely administer an injection with regard to the insertion of the needle and with regard to the application of a desired dosage and a desired dosage rate.

According to an advantageous example, the first retaining section comprises a first snap-fit connector configured to fix the barrel section of the medical syringe perpendicular to the actuating axis.

Advantageously, the barrel section of the syringe is fixed by the first snap-fit connector perpendicular to the actuating axis with a form-closed fit. This protects the syringe from being removed unintentionally.

According to an advantageous example, the second retaining section comprises a second snap-fit connector configured to fix the plunger section of the medical syringe perpendicular to the actuating axis.

Advantageously, the plunger section of the syringe is fixed by the second snap-fit connector perpendicular to the actuating axis with a form-closed fit. This protects the syringe from being removed unintentionally.

According to an advantageous example, the first retaining section comprises a first receiving section, in particular two opposing stop sections, being configured to limit movement of the barrel section of the medical syringe along the actuating axis and relative to the first retaining section.

The first receiving section provides a form-closed fit in the actuating direction. This improves the actuating precision.

According to an advantageous example, the second retaining section comprises a second receiving section, in particular two opposing stop sections, being configured to limit movement of the plunger section of the medical syringe along the actuating axis and relative to the second retaining section.

The second receiving section provides a form-closed fit in the actuating direction. This improves the actuating precision.

According to an advantageous example, the actuator is a manual actuator.

A simple-to-use and cheap apparatus is provided.

According to an advantageous example, the actuator comprises: a manual rotary knob connected to a gear wheel, wherein the manual rotary knob is fixed rotatable to the first retaining section; and a toothed rack of the second retaining section, wherein the gear wheel engages with the toothed rack.

Advantageously, a simple gear-like actuator is provided to enable a precise actuation of the syringe.

According to an advantageous example, the actuator comprises an electric motor, and wherein the apparatus comprises at least one actuator button configured to actuate the electric motor.

Advantageously, an easy-to-use actuator is provided to enable a precise actuation of the syringe.

According to an advantageous example, the actuator comprises: a spindle mounted rotatable to the first retaining section, wherein the spindle is driven by the electric motor, and a spindle nut, which engages with the spindle and which is part of or connected to the second retaining section.

The spindle and the spindle nut translate turning motion into a precise linear motion. Therefore, the syringe is operated via actuator buttons and a corresponding torque is applied to the spindle by the motor.

According to an advantageous example, the apparatus comprises a display, a sensor and a control unit, wherein the control unit is configured to: receive a first sensor signal from the sensor, wherein the first sensor signal represents a first position of the first and second retaining sections to each other; receive a second sensor signal from the sensor, wherein the second sensor signal represents a second position of the first and second retaining sections to each other; determine a volume in dependence on the first sensor signal and in dependence on the second sensor signal; and operate the display to display the determined volume.

Advantageously, the medical practitioner can easily check via the display whether the desired amount of injected fluid or fluid taken off is already reached or not.
- Figures 1 and 2: each depict an example of an apparatus for operating a medical syringe;
- Figure 3: depicts a detail of figure 2;
- Figure 4: depicts a schematical flow diagram;
- Figures 5 and 6: each depict an example of the apparatus for operating the medical syringe;
- Figure 7: depicts a schematical cross-section of the apparatus shown in figures 5 and 6, and
- Figure 8: depicts a schematical arrangement of a sensor unit.

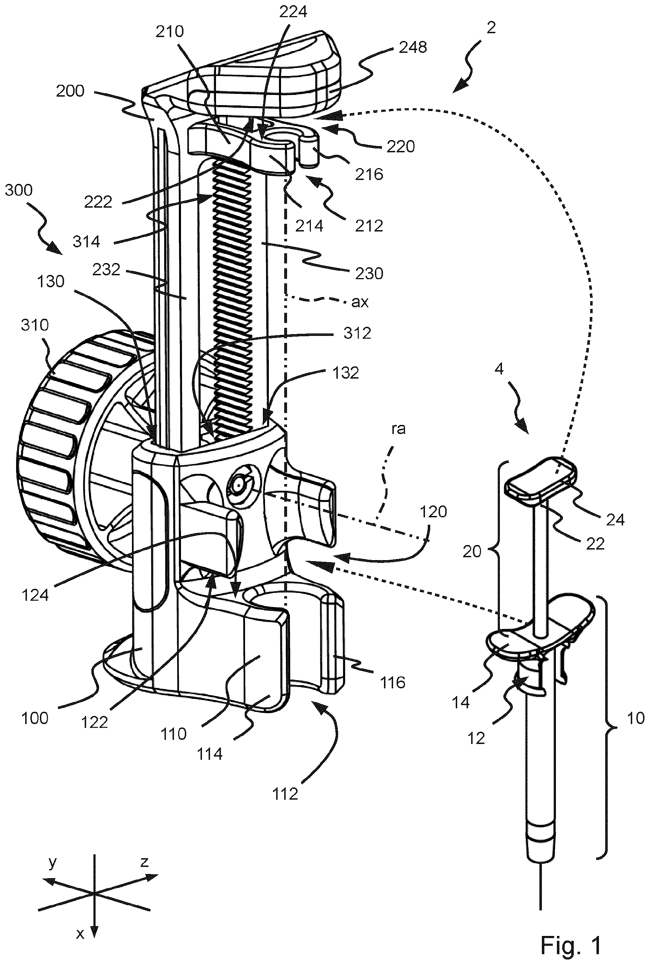

Figure 1 depicts an example of an apparatus 2 for operating a medical syringe 4. For illustration purposes, dimensions of the apparatus 2 and the syringe 4 are not adjusted in the figure.

The syringe 4 comprises, for example, a cylindrical barrel, a piston movable therein and a conical or cylindrical needle adapter. A hollow needle (cannula) is connected to the needle adapter. A plunger is connected to the piston, which is arranged inside the barrel. The plunger comprises a distal actuation section.

A first retaining section 100 is configured to receive and hold removable a barrel section 10 of the medical syringe 4 .

The first retaining section 100 comprises a first snap-fit connector 110 configured to fix the barrel section 10 of the medical syringe 4 perpendicular to the actuating axis ax. Elastic flaps 114 and 116 delimit an inner cylindrical surface. The distal ends of the flaps 114, 116 delimit the inner cylindrical surface with an opening 112, which has a width parallel to the z-axis smaller than the diameter of the inner cylindrical surface. The elastic flaps 114 and 116 enable to clip-in a cylindrical portion 12 of the barrel section 10.

The first retaining section 100 comprises a first receiving section 120, in particular two opposing stop sections 122, 124, being configured to limit movement of the barrel section 10 of the medical syringe 4 along the actuating axis ax and relative to the first retaining section 100. At least one section 14 protruding from the barrel section 10 of the syringe 4 engages in an opening provided by the stop sections 122 and 124. Therefore, the receiving section 120 fixes the section 14 perpendicular to the actuation axis ax with a form-closed fit.

A second retaining section 200 is configured to receive and hold removable a plunger section 20 of the medical syringe 4.

The second retaining section 200 comprises a second snap-fit connector 210 configured to fix the plunger section 20 of the medical syringe 4 perpendicular to the actuating axis ax. Elastic flaps 214 and 216 delimit an inner cylindrical surface. The distal ends of the flaps 214, 216 delimit the inner cylindrical surface with an opening 212, which has a width parallel to the z-axis smaller than the diameter of the inner cylindrical surface. The elastic flaps 214 and 216 enable to clip-in a cylindrical portion 22 of the plunger section 20.

The second retaining section 200 comprises a second receiving section 220, in particular two opposing stop sections 222, 224, being configured to limit movement of the plunger section 20 of the medical syringe 4 along the actuating axis ax and relative to the second retaining section 200. At least one section 24 protruding perpendicularly from a distal end of the plunger section 20 of the syringe 4 engages in an opening provided by the stop sections 222 and 224. Therefore, the second receiving section 220 fixes the section 24 perpendicular to the actuation axis ax with a form-closed fit.

An actuator 300 is configured for axially displacing the first retaining section 100 and the second retaining section 200 in relation to each other and along the actuating axis ax. The actuator 300 is a manual actuator. The actuator 300 comprises a manual rotary knob 310 connected to a gear wheel 312, wherein the manual rotary knob 310 is fixed rotatable along a rotation axis ra to the first retaining section 100. The second retaining section 200 comprises two bars 230 and 232, each bar 230, 232 protruding parallel to the actuating axis ax towards the first retaining section 100. The bars 230, 232 are received in at least one through-hole 130, 132 of the first retaining section 100.

A toothed rack 314 is arranged at the inner side of the bar 230. The gear wheel 312 actuated by the knob 310 engages with the toothed rack 314 in order to move the second retaining section 200 linearly and relative to the first retaining section 100.

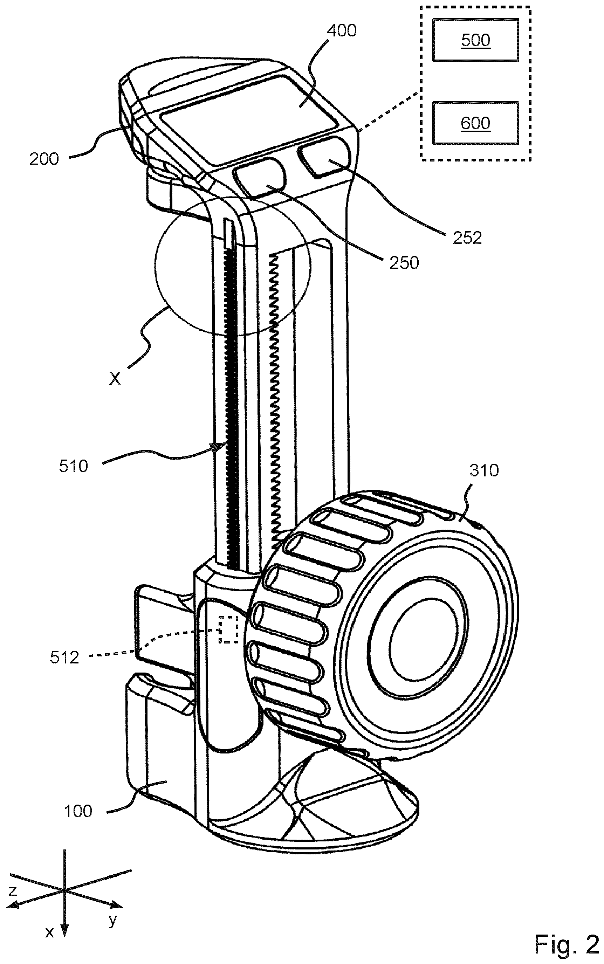

Figure 2 depicts the apparatus of figure 1. The second retaining section 200 comprises a display 400 towards a distal end of the second retaining section 200.

The apparatus 2, in particular the first and/or second retaining section 100, 200, comprises a sensor unit 500 and a control unit 600. The control unit 600 is arranged on at least one printed circuit board, which is arranged inside the housing of the second retaining section 200. Manual buttons 250, 252 are provided near the display 400.

The signals of the buttons 250, 260 are received by the control unit 600. For example, one of the buttons 250, 260 causes the control unit 600 to reset a volume value to zero at the present relative position of the first and second retaining section 100, 200. Upon relative movement of the first and second retaining sections 100, 200, the volume value shown on the display 400 is adapted by the control unit 600.

The second retaining section 200 comprises a battery compartment 248 provided via a drawer for housing a battery or an accumulator.

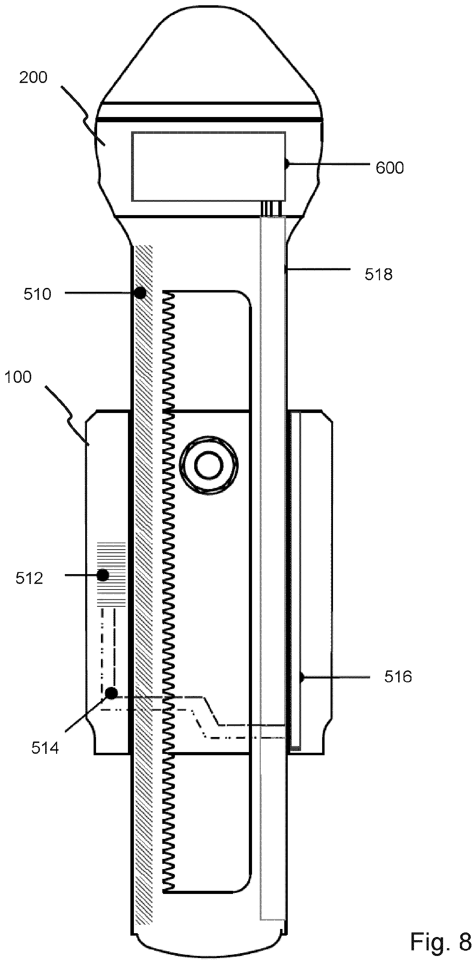

According to an example of the sensor unit 500, a metallic sensor strip 510 comprises a fixed pattern in form of a plurality of consecutive elevations and recesses. The metallic sensor strip 510 is for example made of copper. A capacitive sensor 512 of the first retaining section 100 is configured and arranged to sense the elevations and recesses of the sensor strip 510. The transmission of the signals from the capacitive sensor 512 to the control unit is exemplified in figure 8. Of course, the determination of the relative position between the first and second retaining sections 100 and 200 can be done in other ways.

According to an example, the apparatus 2 does not comprise the display 400, the control unit 600, and the sensor 500.

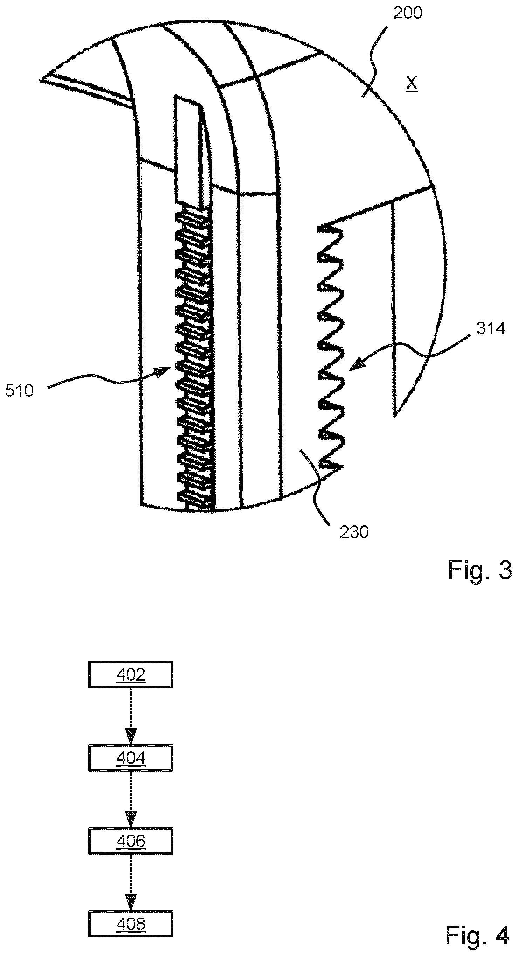

Figure 3 depicts a detail X of figure 2. The bar 230 comprises the sensor strip 510 on its outer side. For example, the sensor strip 510 is divided into 100 gradations with an overall travel of 50mm. According to an example, one of the gradations corresponds to 0.01 ml of fluid. Therefore, each gradation represents the relative position of the first and second retaining sections. The sensor signal representing the former relative position is transmitted to the control unit, which translates the sensor signal to Milliliters being displayed on the display.

According to a further example, the position of the second to the first retaining section is measured via a rotational sensor, measuring a rotation angle of the shaft of the manual rotary knob 310.

Figure 4 depicts a schematical flow diagram to operate the apparatus. The control unit of the apparatus is configured to receive, according to a step 402, a first sensor signal from the sensor, wherein the first sensor signal represents a first position of the first and second retaining sections to each other. The first position is saved in a memory means of the control unit if one of the manual buttons is pressed.

According to a step 404, the control unit receives a second sensor signal from the sensor, wherein the second sensor signal represents a second position of the first and second retaining sections to each other. The second sensor signal is determined continuously and transmitted continuously from the sensor to the control unit.

The control unit determines, according to a step 406, a volume, which for example has already been injected since the first position, in dependence on the first sensor signal and in dependence on the second sensor signal. The control unit is of knowledge of the inner diameter of the syringe. Therefore, the control unit can determine the volume in dependence on a distance between the first and second position and in dependence on the inner diameter.

According to an example, the second retaining section comprises a buzzer or speaker, which is activated to vibrate or emit a sound upon reaching the second position. The vibration or sound represents a tactile signal for signaling to the user that the desired volume has been reached.

The control unit operates, according to a step 408, the display to display the determined volume.

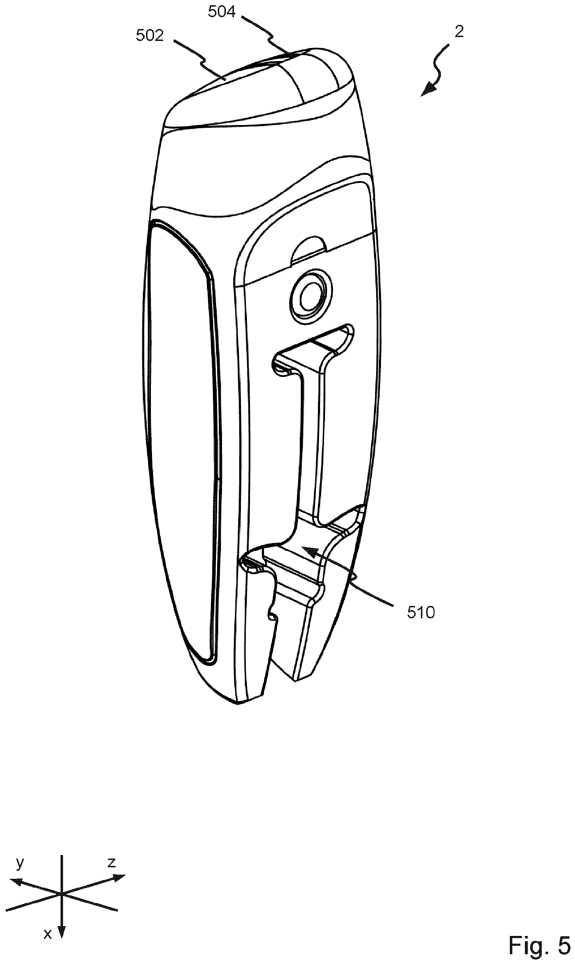

Figure 5 depicts an example of the apparatus 2 for operating the medical syringe. Actuator buttons 502 and 504 are accommodated at a distal end of the apparatus 2 and are configured to actuate the apparatus 2 to retract or push the plunge of the medical syringe. According to an example, the actuator buttons 502 and 504 are capacitive touch buttons.

An opening 510 follows the outer contour of the syringe in a retracted position. The opening 510 is adapted for inserting at least a part of the medical syringe into the apparatus. Reference is made to the other examples in particular with regard to the first and second retaining sections.

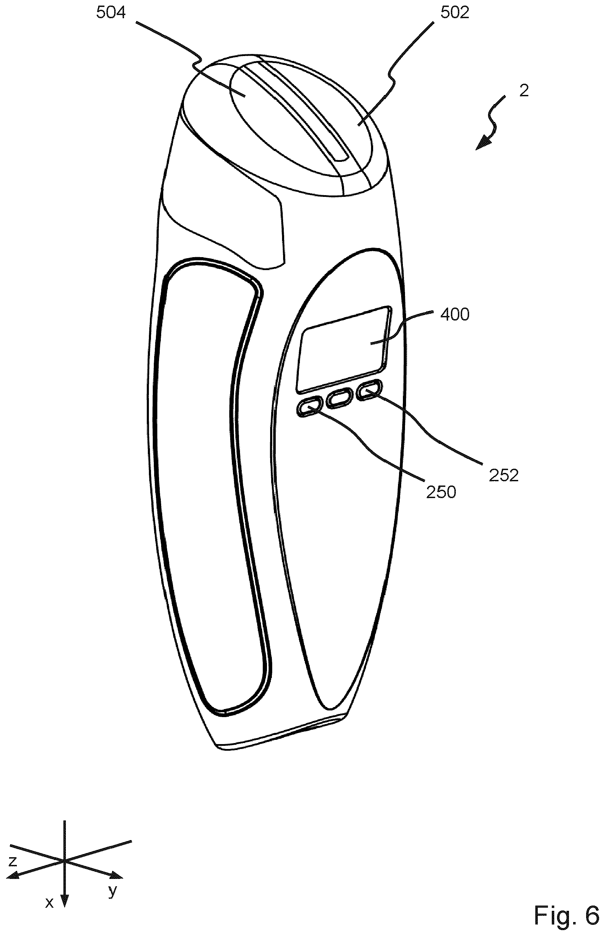

Figure 6 depicts the apparatus of figure 5.

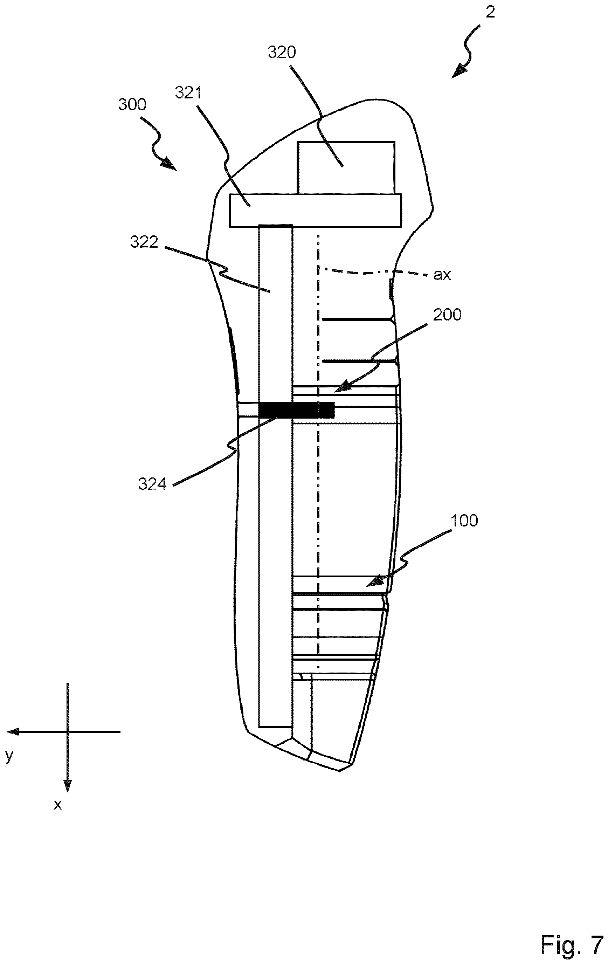

Figure 7 depicts a schematical cross-section of the apparatus 2 shown in figures 5 and 6. The actuator 300 comprises an electric motor 320, for example a micro stepper motor, which is connected via gearbox 321 with a spindle 322. The spindle 322 is mounted rotatable to the first retaining section 100 along an axis parallel to the actuating axis. The spindle 322 is driven by the electric motor 320. The apparatus 2 comprises the actuator buttons being configured to actuate the electric motor 320 in one or another direction. A spindle nut 324, which engages with the spindle 322 and which is part of or connected to the second retaining section 200 moves along the actuating axis ax.

The motor 320 transmits its mechanical moment to the plunger section of the syringe, wherein the plunger section pushes down or up the syringe piston.

According to an example, the control unit of the apparatus is configured to:
- determine a set-point volume, wherein the set-point volume is input via buttons by the user,
- determine, via the sensor, a first position of the first and second retaining sections to each other,
- determine a second position in dependence on the set-point volume and in dependence on the first position, wherein the second position indicates that the set-point volume will have been reached,
- move, via the motor 320, the second retaining section relative to the first retaining section towards the second position,
- determine, via the sensor, that the second position has been reached, and
- stop the motor 320, upon determining that the second position has been reached.

Figure 8 depicts a schematical arrangement of the sensor unit. The capacitive sensor 512 transmits its signal via cables 514 to a first coupling unit 516 of the first retaining section 100. A second coupling unit 518 is arranged at the second retaining section 518. The coupling units 516 and 518 are configured to transmit the signal of the sensor 512 to the control unit 600 arranged in the second retaining section 200. For example, the first coupling unit 516 acts as a transmitter and the second coupling unit 518 acts as a receiver for the sensor data. The first and second coupling unit 516, 518 are used to transmit electric energy to power the sensor 512. For example, magnetic coupling is used for contactless coupling of the first and second coupling units 516 and 518. While the scale rack in the sense of the sensor strip moves up or down the sensor 512 reads the traveled distance in the sense of pulses transmitted to the control unit 600. The control unit 600 converts the signals received from the sensor 512 to liquid volume (ml). For example, the accuracy of the embedded sensor strip is 0.01 mm, which is displayed in the display as 0.01 ml. In other examples, with a different syringe diameter, a different calculation scheme applies.

## Claims

1. An apparatus (2) for operating a medical syringe (4), the apparatus (2) comprising:
a first retaining section (100) configured to receive and hold a barrel section (10) of the medical syringe (4) ;
a second retaining section (200) configured to receive and hold a plunger section (20) of the medical syringe (20); and
an actuator (300) configured to axially displace the first retaining section (100) and the second retaining section (200) in relation to each other and along an actuating axis (ax).

2. The apparatus (2) according to claim 1, wherein the first retaining section (100) comprises a first snap-fit connector (110) configured to fix the barrel section (10) of the medical syringe (4) perpendicular to the actuating axis (ax).

3. The apparatus (2) according to claim 1 or 2, wherein the second retaining section (200) comprises a second snap-fit connector (210) configured to fix the plunger section (20) of the medical syringe (4) perpendicular to the actuating axis (ax).

4. The apparatus (2) according to one of the preceding claims, wherein the first retaining section (100) comprises a first receiving section (120), in particular two opposing stop sections (122, 124), being configured to limit movement of the barrel section (10) of the medical syringe (4) along the actuating axis (ax) and relative to the first retaining section (100).

5. The apparatus (2) according to one of the preceding claims, wherein the second retaining section (200) comprises a second receiving section (220), in particular two opposing stop sections (222, 224), being configured to limit movement of the plunger section (20) of the medical syringe (4) along the actuating axis (ax) and relative to the second retaining section (200).

6. The apparatus (2) according to one of the preceding claims, wherein the actuator (300) is a manual actuator.

7. The apparatus (2) according to claim 6, wherein the actuator (300) comprises:
a manual rotary knob (310) connected to a gear wheel (312), wherein the manual rotary knob (310) is fixed rotatable to the first retaining section (100); and
a toothed rack (314) of the second retaining section (200), wherein the gear wheel (312) engages with the toothed rack (314).

8. The apparatus (2) according to one of the preceding claims, wherein the actuator (300) comprises an electric motor (320), and wherein the apparatus (2) comprises at least one actuator button (502; 504) configured to actuate the electric motor (320).

9. The apparatus (2) according to claim 8, wherein the actuator (300) comprises:
a spindle (322) mounted rotatable to the first retaining section (100), wherein the spindle (322) is driven by the electric motor (320), and
a spindle nut (324), which engages with the spindle (322) and which is part of or connected to the second retaining section (200).

10. The apparatus (2) according to one of the preceding claims, wherein the apparatus (2) comprises a display (400), a sensor (500) and a control unit (600), wherein the control unit (600) is configured to:
receive (402) a first sensor signal from the sensor (400), wherein the first sensor signal represents a first position of the first and second retaining sections (100, 200) to each other;
receive (404) a second sensor signal from the sensor (400), wherein the second sensor signal represents a second position of the first and second retaining sections (100, 200) to each other;
determine (406) a volume in dependence on the first sensor signal and in dependence on the second sensor signal; and
operate (408) the display (400) to display the determined volume.
